# EUROPEAN PATENT APPLICATION

(11) **EP 4 292 605 A1**
(43) Date of publication of application: **20.12.2023**
(21) Application number: 22778898.1
(22) Date of filing: 29.03.2022
(51) Int. Cl.: A61K 38/35, A61K 9/08, A61K 9/48, A61P 19/02

(54) **DRUG CONTAINING ADRENOCORTICOTROPIC HORMONE OR DERIVATIVE THEREOF AND USE THEREOF**

(30) Priority: 31.03.2021 CN 202110345922
(71) Applicant: Zhang, Jing, Gusu District Suzhou, Jiangsu 215000 (CN)
(72) Inventor: ZHANG, Jing, Suzhou, Jiangsu 215000 (CN); JIN, Wenbo, Suzhou, Jiangsu 215000 (CN)
(74) Representative: Nederlandsch Octrooibureau
(86) International application number: PCT/CN2022/083523
(87) International publication number: WO 2022/206716

(57) **Abstract**

The present invention provides use of an oral pharmaceutical formulation containing adrenocorticotropic hormone or its derivatives in the preparation of medications for the treatment of arthritis. In particular, the oral pharmaceutical formulation is used for the treatment of gout caused by a high uric acid level.

## Description

### TECHNICAL FIELD

The present invention belongs to the field of biomedical technology and particularly relates to a medication containing adrenocorticotropic hormone or its derivatives and use thereof.

### BACKGROUND

Adrenocorticotropic hormone (ACTH) is a peptide hormone secreted by the pituitary gland in vertebrates, which stimulates tissue proliferation in the adrenal cortex and the production and secretion of corticosteroid. The production and secretion of adrenocorticotropic hormone is directly regulated by the hypothalamic adrenocorticotropic hormone releasing factor. Excessive secretion of corticosteroids can in turn affect the pituitary and hypothalamus and weaken their activity. Adrenocorticotropic hormone stimulates the development and function of the adrenal cortex, mainly acting on the zona fasciculata of the adrenal cortex and stimulating the secretion of glucocorticosteroids. Adrenocorticotropic hormone also plays a role in regulating antibody production through the adrenal cortex.

Adrenocorticotropic hormone is a peptide drug, which is easily degraded by gastrointestinal digestive enzymes and other enzymes, thus it has not been possible to achieve oral administration. Clinically applied pharmaceutical formulations containing adrenocorticotropic hormone are injectable preparations. Adrenocorticotropic hormone for injection (corticotropin for injection) is indicated for use in collagenous disorders such as active rheumatism, rheumatoid arthritis, and erythematous lupus; it is also used in allergic disorders such as severe bronchial asthma and severe dermatitis, and in acute leukaemia and Hodgkin's disease, etc.

The search in the prior art has not revealed any pharmaceutical formulations of adrenocorticotropic hormone for oral administration or new uses of such oral pharmaceutical formulation.

### SUMMARY OF THE INVENTION

For the above reasons, the present inventors have obtained an oral pharmaceutical formulation containing adrenocorticotropic hormone or its derivatives and its new use through a lot of creative research. Studies have shown that an oral pharmaceutical formulation containing adrenocorticotropic hormone or its derivatives is effective in the treatment of arthritis, including gout caused by a high uric acid level.

The present invention is realized by the following technical solution.

Use of an oral pharmaceutical formulation containing adrenocorticotropic hormone or its derivatives in the preparation of medicaments for the treatment of arthritis.

Especially for acute gout caused by a high uric acid level, it has a good therapeutic effect.

The oral pharmaceutical formulation comprises a surfactant, an acrylic polymer, chitin or its derivatives and a metal ion chelating agent as excipients.

The oral pharmaceutical formulation comprises a surfactant, chitin or its derivatives and a metal ion chelating agent as excipients.

The surfactant is one or more selected from a group consisting of an anionic surfactant and a non-ionic surfactant.

The acrylic polymer is one or more selected from a group consisting of Carbomer, Carbomer 910, Carbomer 934, and Carbomer 934P.

Chitin or its derivatives is one or more selected from a group consisting of chitin, chitosan, carboxymethyl chitosan, acylated chitosan, alkylated chitosan, hydroxylated chitosan, chitosan quaternary ammonium salts, chitosan oligosaccharides and chitosan sulphates.

The metal ion chelating agent is one or more selected from a group consisting of citric acid or its salts, tartaric acid or its salts, malic acid or its salts, maleic acid or its salts, gluconic acid or its salts, ethylenediaminetetraacetic acid or its salts, aminotriacetic acid or its salts, and diethylenetriaminepentaacetic acid or its salts.

The weight ratio of the surfactant, the acrylic polymer, chitin or its derivative and the metal ion chelating agent is 19-21 : 6-7 : 6-7 : 60-70.

The weight ratio of the surfactant, chitin or its derivative and the metal ion chelating agent is 19-21 : 6-7 : 60-70.

The weight ratio of ACTH (or its derivatives), the surfactant, the acrylic polymer, chitin or its derivatives and the metal ion chelating agent is 1.5-2.5 : 19-21 : 6-7: 6-7 : 60-70.

The weight ratio of ACTH (or its derivatives), the surfactant, chitin or its derivatives and the metal ion chelating agent is 1.5-2.5 : 19-21 : 6-7 : 60-70.

The weight ratio of ACTH (or its derivatives), the surfactant, the acrylic polymer, chitin or its derivatives and the metal ion chelating agent is 0.20-0.30 : 19-21 : 6-7 : 6-7 : 60-70.

The weight ratio of ACTH (or its derivatives), the surfactant, chitin or its derivatives and the metal ion chelating agent is 0.20-0.30 : 19-21 : 6-7 : 60-70.

The weight ratio of ACTH (or its derivatives), the surfactant, the acrylic polymer, chitin or its derivatives and the metal ion chelating agent is 0.25-2.0 : 19-21 : 6-7 : 6-7 : 60-70.

The weight ratio of ACTH (or its derivatives), the surfactant, chitin or its derivatives and the metal ion chelating agent is 0.25-2.0 : 19-21 : 6-7 : 60-70.

Among the above weight ratios, the preferred weight ratio of ACTH (or its derivatives), the surfactant, chitin or its derivatives and the metal ion chelating agent is 0.25-2.0 : 19-21 : 6-7 : 60-70.

Adrenocorticotropic hormone or its derivatives is present at a concentration of 0.05 mg/ml or higher in a solution formulation or in a content of 0.4 mg or higher in a capsule formulation.

The concentration in a solution formulation means that it is greater than or equal to 0.05 mg/ml to a saturated concentration in an aqueous solution;

The content in a capsule formulation means a loading of greater than or equal to 0.4 mg per capsule up to the maximum loading of one capsule.

Adrenocorticotropic hormone or its derivatives is selected from a group consisting of ACTH (1-39), ACTH (1-24) and ACTH (1-17).

Adrenocorticotropic hormone (ACTH) described herein is a peptide hormone secreted by the pituitary gland in vertebrates, which stimulates tissue proliferation in the adrenal cortex and the production and secretion of corticosteroid. ACTH contains 39 amino acids, of which amino acids 1 to 24 are essential amino acids for biological activity, and amino acids 25 to 39 are protective amino acids.

At present, products containing "adrenocorticotropic hormone (ACTH)" as an active ingredient in the clinic are ACTH for injection; whereas the present invention provides a pharmaceutical composition for oral administration containing adrenocorticotropic hormone (ACTH) or its derivatives as an active ingredient for the treatment of arthritis including gout.

The adrenocorticotropic hormone derivatives described herein include, but are not limited to, ACTH (1-24), which is the N-terminal 24 peptide of adrenocorticotropic hormone. ACTH (1-24) has the same physiological activity as ACTH because amino acids 1 to 24 are the essential amino acids for biological activity. Therefore, when it is made into an oral pharmaceutical formulation, it should have the same effect on arthritis, including gout caused by a high uric acid level.

Arthritis broadly refers to inflammatory diseases occurring in joints and surrounding tissues of the body, which are caused by inflammation, infection, degeneration, trauma, or other factors, and can be classified into dozens of categories. Clinical manifestations include redness, swelling, heat, pain, dysfunction and joint deformity of the joints, which can lead to joint disability and affect the quality of life of patients in severe cases. The etiology of arthritis is very complicated, mainly related to autoimmune reactions, infections, metabolic disorders, trauma and degenerative diseases. According to the etiology, arthritis can be classified into osteoarthritis, rheumatoid arthritis, rheumatoid arthritis and gouty arthritis. There are more than 100 million patients with arthritis in China, and the number is growing, especially gouty arthritis.

Gout is a common and complicated arthritis, which can occur at any age, and it is more common in men than in women. Gout patients often experience sudden joint pain at night, and severe pain, edema, redness and inflammation quickly appear in the joint area, which gradually decreases until it disappears, lasting for several days to weeks. When the pain suddenly strikes (acute gout), patients wake up from deep sleep in the middle of the night because of the pain. Some patients describe the pain as feeling as if their big toe is on fire. The most common joint involved is the big toe (medical term: first metatarsal bone), but the disease is not limited to this joint, and the joints of the hands, knees and elbows are also common. The diseased joints eventually become red, swollen and inflamed, and edema will soften tissues, limit activities, and eventually affect daily life. These symptoms can appear repeatedly.

The anionic surfactant, nonionic surfactant, etc. according to the present invention are commercially available.

The acrylic polymer according to the present invention includes Carbomer, Carbomer 910, Carbomer 934 and Carbomer 934P, which are commercially available.

Chitin or its derivatives according to the present invention comprise chitin, chitosan, carboxymethyl chitosan, acylated chitosan, alkylated chitosan, hydroxylated chitosan, chitosan quaternary ammonium salts, chitosan oligosaccharides and chitosan sulphates, which are commercially available.

The metal ion chelating agent according to the present invention comprises citric acid or its salts, tartaric acid or its salts, malic acid or its salts, maleic acid or its salts, gluconic acid or its salts, ethylenediaminetetraacetic acid or its salts, aminotriacetic acid or its salts, and diethylenetriaminepentaacetic acid or its salts, which are commercially available.

The present invention contributes to the prior art by forming a new composition using ACTH or its derivatives with a surfactant, chitin or its derivatives and a metal ion chelating agent, which is used for treating arthritis, especially gout caused by a high uric acid level when administered orally.

The present invention contributes to the prior art by forming a new composition using ACTH or its derivatives with a surfactant, an acrylic polymers, chitin or its derivatives and a metal ion chelating agent, which is used for treating arthritis, especially gout caused by a high uric acid level when administered orally.

The preferred oral pharmaceutical composition for treating gout is a new composition formed by ACTH or its derivatives with a surfactant, chitin or its derivative and a metal ion chelating agent for the reason that: on the basis of selecting a composition with good therapeutic effect on arthritis (including gout caused by a high uric acid level) after the combination of these three components with ACTH or its derivatives, the composition has the advantages of reducing the components of the composition, having better safety, reducing the cost, reducing the burden of patients and the like.

### MODE OF CARRYING OUT THE INVENTION

The technical solutions of the present invention are described below by way of specific experimental examples, but the protection scope of the present invention is not limited thereto.

What is described in the experimental examples of the specification is only an enumeration of the realization forms of the concept of the invention, and the protection scope of the invention should not be regarded as limited to the specific forms described in the experimental examples, and the protection scope of the invention also extends to equivalent technical means that those skilled in the art can think of according to the concept of the invention. Notwithstanding the following description of embodiments of the present invention, the invention is not limited to the specific embodiments and fields of application described above, and the specific embodiments described below are merely illustrative and instructive, and not limiting.

### Experimental Examples

The following experiments of the invention are decisive experiment summarized by the present inventors on the basis of many creative experiments and the technical solution protected by the present invention. For the quantitative experiments in the following experimental examples, three repeated experiments were performed, and the data was the mean of the three replicate experiments or the mean ± standard deviation.

### Experiment 1: Test of the effect on joint swelling (gout) caused by a high uric acid level in a mouse model

The groupings were as follows:
1. Group of four-component drug: a solution formulation containing 0.5 mg/ml of adrenocorticotropic hormone, 4 mg/ml of sodium dodecyl sulphate, 1.3 mg/ml of chitosan, and 13 mg/ml of sodium citrate, was administered via the small intestine at a volume of 4 ml/kg.
2. Low dose group of five-component drug: a solution formulation containing 0.05 mg/ml of adrenocorticotropic hormone, 4 mg/ml of sodium dodecyl sulphate, 1.3 mg/ml of Carbomer, 1.3 mg/ml of chitosan, and 13 mg/ml of sodium citrate, was administered via the small intestine at a volume of 4 ml/kg.
3. High dose group of five-component drug: a solution formulation containing 0.5 mg/ml of adrenocorticotropic hormone, 4 mg/ml of sodium dodecyl sulphate, 1.3 mg/ml of Carbomer, 1.3 mg/ml of chitosan, and 13 mg/ml of sodium citrate, was administered via the small intestine at a volume of 4 ml/kg.
4. Group of single active ingredient drug: a solution formulation containing 0.5 mg/ml of adrenocorticotropic hormone, was administered via the small intestine at a volume of 4ml/kg.
5. Group of four-component excipients: a solution formulation containing 4 mg/ml of sodium dodecyl sulphate, 1.3 mg/ml of Carbomer, 1.3 mg/ml of chitosan, and 13 mg/ml of sodium citrate, was administered via the small intestine at a volume of 4 ml/kg.
6. Positive control group (no drug administered): saline was administered via the small intestine at a volume of 4 ml/kg.

Experimental procedure: C57 male mice aged 6 to 8 weeks were anesthetized via a combination of chloral hydrate and xylazine, and 50 µl of 20 mg/ml sodium urate was injected into the right hind foot and 50 µl of saline was injected into the left hind foot. Half an hour later, depending on the grouping, 4 ml/kg of the test drug was administered to the small intestines of 6 mice in each group through a small opening in the abdominal cavity. The diameters of the right and left hind feet were measured at 0, 6, 12 and 24 hours using the administration of sodium urate as 0 hour. Each animal was measured three times at each time point to minimize operational error. The effectiveness of the drug was evaluated by comparing the ratio of the diameter of right hind foot to the diameter of left hind foot.

**Table 1 Effect on ioint swelling (gout) caused by a high uric acid level**

| Groups | Diameter of right hind foot/Diameter of left hind foot | | | |
|---|---|---|---|---|
| | 0 h | 6h | 12 h | 24 h |
| Group of four-component drug | 1.01±0.04 | 1.10±0.13^{a} | 1.16±0.19^{a} | 1.11±0.15^{a} |
| Low dose group of five-component drug | 1.00±0.08 | 1.12±0.16^{a} | 1.27±0.16^{a} | 1.19±0.22 |
| High dose group of five-component drug | 0.98±0.07 | 1.03±0.18^{b} | 1.09±0.13^{b} | 1.06±0.20^{b} |
| Group of single active ingredient drug | 1.02±0.09 | 1.15±0.16 | 1.32±0.18 | 1.22±0.12 |
| Group of four-component excipients | 1.01±0.03 | 1.18±0.15 | 1.31±0.14 | 1.21±0.15 |
| Positive control group | 0.99±0.09 | 1.18±0.18 | 1.35±0.19 | 1.24±0.20 |

| | | | | |
|---|---|---|---|---|
| a: p value<0.05 vs Positive control group (no drug administered); b: p value<0.01 vs Positive control group (no drug administered) | | | | |

Experiment conclusion: the experiments showed (as shown in Table 1) that adrenocorticotropic hormone, when prepared into an oral pharmaceutical formulation, had a good alleviating effect on joint swelling (gout) caused by a high uric acid levels (acute gout) in mice. Studies show that the group of four-component drug (the combination of ACTH with sodium dodecyl sulphate, chitosan, and sodium citrate) has a sustained 24-hour effect on joint swelling caused by a high uric acid level; similarly, the high dose group of five-component drug has a sustained 24-hour effect.

### Experiment 2: Test of the effect on joint swelling (gout) caused by a high uric acid level in a beagle dog model

The groupings were as follows:
1. Group of five-component drug: No. 3 enteric-coated capsule containing 4 mg of adrenocorticotropic hormone, 40 mg of sodium dodecyl sulfate, 13 mg of Carbomer, 13 mg of chitosan, and 130 mg of sodium citrate was taken orally.
2. Group of single active ingredient drug: No. 3 enteric-coated capsule containing 4 mg of adrenocorticotropic hormone was taken orally.
3. Positive control group (no drug administered): Empty No. 3 enteric-coated capsule taken orally.

Experimental procedure: male beagle dogs aged 6 to 8 weeks were given different test drug orally, depending on the grouping. After 1 h, the animals were immobilized and 1 ml of 20 mg/ml sodium urate was injected into the right hind foot and 1 ml of saline into the left hind foot of each beagle dog. There were 3 animals in each group. The diameters of the right and left hind feet were measured at 0, 6, 12 and 24 hours using the administration of sodium urate as 0 hour. Each animal was measured three times at each time point to minimize operational error. The effectiveness of the drug was evaluated by comparing the ratio of the diameter of right hind foot to the diameter of left hind foot.

**Table 2: Effect on joint swelling (gout) caused by a high uric acid level in a beagle dog model**

| Groups | Diameter of right hind foot/Diameter of left hind foot | | | |
|---|---|---|---|---|
| | 0 h | 6h | 12h | 24 h |
| Group of five-component drug | 0.99±0.08 | 1.05±0.18^{b} | 1.10±0.13^{b} | 1.07±0.19^{b} |
| Group of single active ingredient drug | 1.00±0.10 | 1.19±0.16 | 1.38±0.18 | 1.24±0.16 |
| Positive control group (no drug administered) | 1.01±0.09 | 1.21±0.15 | 1.37±0.18 | 1.25±0.19 |

| | | | | |
|---|---|---|---|---|
| a: p value<0.05 vs Positive control group (no drug administered); b: p value<0.01 vs Positive control group (no drug administered) | | | | |

Experiment results: the experiments showed (as shown in Table 2) that adrenocorticotropic hormone, when prepared into an oral pharmaceutical formulation, had a good alleviating effect on joint swelling (gout) caused by a high uric acid level in beagle dogs.

### Preparation Examples

### Example 1

4 g of adrenocorticotropic hormone, 40 g of sodium dodecyl sulfate, 13 g of Carbomer, 13 g of chitosan, and 130 g of sodium citrate were used as raw materials.

The preparation procedure included the steps of pulverizing and sieving sodium dodecyl sulfate, Carbomer, chitosan and sodium citrate, respectively, then mixing the resulting powders with adrenocorticotropic hormone, and encapsulating the resulting mixture in enteric-coated capsules.

### Example 2

4 g of adrenocorticotropic hormone, 40 g of sodium dodecyl sulfate, 13 g of Carbomer 934P, 13 g of carboxymethyl chitosan, and 130 g of sodium malate were used as raw materials.

The preparation procedure included the steps of pulverizing and sieving sodium dodecyl sulfate, Carbomer 934P, carboxymethyl chitosan and sodium malate, respectively, then mixing the resulting powders with adrenocorticotropic hormone, and encapsulating the resulting mixture in enteric-coated capsules.

### Example 3

4 g of adrenocorticotropic hormone, 40 g of sodium dodecyl sulfate, 13 g of Carbomer 934, 13 g of acylated chitosan, and 130 g of sodium malate were used as raw materials.

The preparation procedure included the steps of pulverizing and sieving sodium dodecyl sulfate, Carbomer 934, acylated chitosan and sodium malate, respectively, then mixing the resulting powders with adrenocorticotropic hormone, and encapsulating the resulting mixture in enteric-coated capsules.

### Example 4

5 g of adrenocorticotropic hormone, 400 g of sodium dodecyl sulfate, 130 g of Carbomer, 130 g of chitosan, and 1300 g of sodium citrate were used as raw materials.

The preparation procedure included the steps of pulverizing and sieving sodium dodecyl sulfate, Carbomer, chitosan and sodium citrate, respectively, then mixing the resulting powders with adrenocorticotropic hormone, and encapsulating the resulting mixture in enteric-coated capsules.

### Example 5

4 g of adrenocorticotropic hormone, 40 g of sodium dodecyl sulfate, 13 g of chitosan, and 130 g of sodium citrate were used as raw materials.

The preparation procedure included the steps of pulverizing and sieving sodium dodecyl sulfate, chitosan and sodium citrate, respectively, then mixing the resulting powders with adrenocorticotropic hormone, and encapsulating the resulting mixture in enteric-coated capsules.

### Example 6

4 g of adrenocorticotropic hormone, 40 g of sodium dodecyl sulfate, 13 g of carboxymethyl chitosan, and 130 g of sodium malate were used as raw materials.

The preparation procedure included the steps of pulverizing and sieving sodium dodecyl sulfate, carboxymethyl chitosan and sodium malate, respectively, then mixing the resulting powders with adrenocorticotropic hormone, and encapsulating the resulting mixture in enteric-coated capsules.

### Example 7

4 g of adrenocorticotropic hormone, 40 g of sodium dodecyl sulfate, 13 g of acylated chitosan, and 130 g of sodium malate were used as raw materials.

The preparation procedure included the steps of pulverizing and sieving sodium dodecyl sulfate, acylated chitosan and sodium malate, respectively, then mixing the resulting powders with adrenocorticotropic hormone, and encapsulating the resulting mixture in enteric-coated capsules.

### Example 8

0.5 g of adrenocorticotropic hormone, 40 g of sodium dodecyl sulfate, 13 g of chitosan, and 130 g of sodium citrate were used as raw materials.

The preparation procedure included the steps of pulverizing and sieving sodium dodecyl sulfate, chitosan and sodium citrate, respectively, then mixing the resulting powders with adrenocorticotropic hormone, and encapsulating the resulting mixture in enteric-coated capsules.

### Example 9

1 g of adrenocorticotropic hormone, 40 g of sodium dodecyl sulfate, 13 g of chitosan, and 130 g of sodium citrate were used as raw materials.

The preparation procedure included the steps of pulverizing and sieving sodium dodecyl sulfate, chitosan and sodium citrate, respectively, then mixing the resulting powders with adrenocorticotropic hormone, and encapsulating the resulting mixture in enteric-coated capsules.

Example 10 3 g of adrenocorticotropic hormone, 40 g of sodium dodecyl sulfate, 13 g of chitosan, and 130 g of sodium citrate were used as raw materials.

The preparation procedure included the steps of pulverizing and sieving sodium dodecyl sulfate, chitosan and sodium citrate, respectively, then mixing the resulting powders with adrenocorticotropic hormone, and encapsulating the resulting mixture in enteric-coated capsules.

The Examples include, but are not limited to, the above Preparation Examples.

Under the teaching of this specification, those skilled in the art can make many other forms without departing from the scope of protection of the present invention, and all these forms fall within the scope of protection of the present invention.

## Claims

1. Use of an oral pharmaceutical formulation containing adrenocorticotropic hormone or its derivatives in preparation of medications for treatment of arthritis.

2. Use according to claim 1, wherein the arthritis includes gout caused by a high uric acid level.

3. Use according to claim 1, wherein the oral pharmaceutical formulation comprises a surfactant, an acrylic polymer, chitin or its derivative and a metal ion chelating agent as excipients.

4. Use according to claim 1, wherein the oral pharmaceutical formulation comprises a surfactant, chitin or its derivative and a metal ion chelating agent as excipients.

5. Use according to claim 3 or 4, wherein the surfactant is one or more selected from a group consisting of an anionic surfactant and a non-ionic surfactant.

6. Use according to claim 3, wherein the acrylic polymer is one or more selected from a group consisting of Carbomer, Carbomer 910, Carbomer 934, and Carbomer 934P.

7. Use according to claim 3 or 4, wherein chitin or its derivatives is one or more selected from a group consisting of chitin, chitosan, carboxymethyl chitosan, acylated chitosan, alkylated chitosan, hydroxylated chitosan, chitosan quaternary ammonium salts, chitosan oligosaccharides and chitosan sulphates.

8. Use according to claim 3 or 4, wherein the metal ion chelating agent is one or more selected from a group consisting of citric acid or its salts, tartaric acid or its salts, malic acid or its salts, maleic acid or its salts, gluconic acid or its salts, ethylenediaminetetraacetic acid or its salts, aminotriacetic acid or its salts, and diethylenetriaminepentaacetic acid or its salts.

9. Use according to claim 3, wherein a weight ratio of the surfactant, the acrylic polymer, chitin or its derivatives and the metal ion chelating agent is 19-21 : 6-7 : 6-7 : 60-70.

10. Use according to claim 4, wherein a weight ratio of the surfactant, chitin or its derivatives and the metal ion chelating agent is 19-21 : 6-7 : 60-70.

11. Use according to claim 1, wherein adrenocorticotropic hormone or its derivatives is present at a concentration of 0.05 mg/ml or higher in a solution formulation or in a content of 0.4 mg or higher in a capsule formulation.

12. Use according to claim 1, wherein adrenocorticotropic hormone or its derivatives is selected from a group consisting of ACTH (1-39), ACTH (1-24) and ACTH (1-17).
